# EUROPEAN PATENT APPLICATION

(11) **EP 2 770 325 A1**
(43) Date of publication of application: **27.08.2014**
(21) Application number: 13158940.0
(22) Date of filing: 13.03.2013
(51) Int. Cl.: G01N 33/68, C12Q 1/68

(54) **Mutations of DEPDC5 for diagnosing epilepsic diseases, disorders or conditions**

(30) Priority: 26.02.2013 US 201361769345 P
(71) Applicant: Institut du Cerveau et de la Moelle Épinière-ICM, 75013 Paris (FR); Centre National de la Recherche Scientifique (CNRS), 75016 Paris (FR); Institut National de la Santé et de la Recherche Médicale (INSERM), 75654 Paris Cedex 13 (FR); Assistance Publique-Hôpitaux de Paris (APHP), 75001 Paris (FR); Université Pierre et Marie Curie (Paris 6), 75005 Paris (FR)
(72) Inventor: Baulac, Stéphanie, 75005 Paris (FR); Leguern, Éric, 93800 Épinay-sur-Seine (FR)
(74) Representative: Icosa

(57) **Abstract**

The present invention relates to a method for diagnosing an epileptic disease, disorder or condition in a subject, or determining a predisposition of a subject to an epileptic disease, disorder or condition or determining an increased risk of a subject of developing an epileptic disease, disorder or condition, wherein said method comprises determining the status of the *DEPDC5* gene of the subject.

## Description

### FIELD OF INVENTION

The present invention relates to the diagnosis of epileptic diseases, disorders or conditions. More specifically, the present invention relates to a method for diagnosing epileptic diseases, disorders or conditions in a subject in need thereof, said method comprising determining the status of the *DEPDC5* gene of the subject.

### BACKGROUND OF INVENTION

Epilepsy is a frequent neurological disorder characterized by spontaneous, recurrent seizures. Epilepsies affect about 3% of the population at some time in their lives, representing about 50 million people worldwide. Epilepsy becomes more common as people age, and onset of new cases occurs most frequently in infants and the elderly.

Focal epileptic seizures are thought to originate within networks limited to one hemisphere. Several autosomal dominant, non-lesional, focal epilepsies have been described as specific age-related and electro-clinical syndromes: autosomal dominant nocturnal frontal lobe epilepsy (ADNFLE), familial temporal lobe epilepsy (FTLE), including mesial and lateral forms (also termed autosomal dominant epilepsy with auditory features (ADEAF)), and familial focal epilepsy with variable foci (FFEVF), characterized by focal seizures that are initiated in distinct cortical regions in different family members.

Some mutations have been previously identified in familial forms of epilepsies, especially mutations in ion channels: WO01/098486 describes mutations in genes *GABRG2* and *GABRD* encoding subunits of the GABA receptor; WO02/50096 describes mutations in the *SCN1A* gene, encoding the alphal-subunit of the brain voltage-gated sodium channel associated with epilepsy, in particular with generalized epilepsy with febrile seizures plus (GEFS+); US2010/136623 describes mutations of the *CHRNA4* and *CHRNB2* genes, encoding subunits of the neuronal nicotinic acetylcholine receptor in patients with ADNFLE; US7,528,093 describes mutations in the *SCN1A, SCN2A* and *SCN3A* loci, encoding proteins for the alpha subunit isoforms of the neuronal sodium channel Type I in patients with idiopathic generalized epilepsy (IGE); and Heron et al describes a mutation in the *KCNT1* gene, encoding a sodium-gated potassium channel subunit in patients with ADNFLE (Heron et al, Nat Genet, 2012, 44:1188-90).

Moreover, some mutations in genes not encoding ion channel or transmitter receptor subunits were identified. For example, US2011/287418 describes the genes *EFHC1* (encoding myoclonin 1) and *EFHC1α* (encoding myoclonin 2) as diagnostic markers for juvenile myoclonic epilepsy (JME). Furthermore, Kalachikov et al describes mutations in the *LGI1* gene in ADEAF patients (Kalachikov et al, Nat Genet, 2002, 30(3):335-341).

However, to the Applicant's knowledge, no causal genes have been identified yet for familial focal epilepsy with variable foci (FFEVF). Searching such a causal gene, the Applicant herein identified mutations in the *DEPDC5* gene, and provided clear evidence that mutations of the *DEPDC5* gene are a major cause in a broad spectrum of autosomal dominant focal epilepsies, including ADNFLE, FTLE and FFEVF.

### SUMMARY

The present invention thus relates to a method for diagnosing an epileptic disease, disorder or condition in a subject, or determining a predisposition of a subject to an epileptic disease, disorder or condition or determining an increased risk of a subject of developing an epileptic disease, disorder or condition, wherein said method comprises determining the status of the *DEPDC5* gene of the subject.

In one embodiment, the method comprises detecting at least one mutation in the *DEDPC5* gene sequence in a sample from the subject. In one embodiment, the at least one mutation is a frameshift mutation, preferably a frameshift mutation introducing a premature stop codon within the sequence of the *DEPDC5* gene; a missense mutation, a nonsense mutation or a gene rearrangement. In one embodiment, the method of the invention comprises detecting at least one mutation listed in Table 2 in a sample from the subject.

In one embodiment, the method comprises assessing the expression level of *DEPDC5* in a sample from the subject, and comparing said expression level with a reference expression value. In one embodiment, the expression level of *DEPDC5* is measured at the protein level. In another embodiment, the expression level of *DEPDC5* is measured at the RNA level. In one embodiment, a reduced expression of *DEPDC5* in the sample from the subject is indicative of the affection of the subject with an epileptic disease, disorder or condition; and/or of the presence of an epileptic disease, disorder or condition in the subject; and/or of an increased risk of the subject to develop an epileptic disease, disorder or condition; and/or of a predisposition of the subject with an epileptic disease, disorder or condition. In one embodiment, the reference expression value is measured in a reference population, preferably a reference population comprising substantially healthy subjects.

In one embodiment of the invention, the sample is a body fluid sample, preferably a blood sample.

In one embodiment, the subject presents a familial history of an epileptic disease, disorder or condition. In one embodiment, an onset of an epileptic disease, disorder or condition has been diagnosed in the subject. In another embodiment, no onset of an epileptic disease, disorder or condition has been diagnosed in the subject.

In one embodiment, the epileptic disease, disorder or condition is selected from the list comprising benign familial neonatal epilepsy (BFNE), early myoclonic encephalopathy (EME), Ohtahara syndrome, epilepsy of infancy with migrating focal seizures, West syndrome, Myoclonic epilepsy in infancy (MEI), benign infantile epilepsy, benign familial infantile epilepsy, Dravet syndrome, myoclonic encephalopathy in non-progressive disorders, febrile seizures plus (FS+), Panayiotopoulos syndrome, epilepsy with myoclonic atonic seizures, benign epilepsy with centrotemporal spikes (BECTS), autosomal-dominant nocturnal frontal lobe epilepsy (ADNFLE), late onset childhood occipital epilepsy, epilepsy with myoclonic absences, Lennox-Gastaut syndrome, epileptic encephalopathy with continuous spike-and-wave during sleep (CSWS), Landau-Kleffner syndrome (LKS), childhood absence epilepsy (CAE), juvenile absence epilepsy (JAE), juvenile myoclonic epilepsy (JME), epilepsy with generalized tonic-clonic seizures alone, progressive myoclonus epilepsies (PME), autosomal dominant epilepsy with auditory features (ADEAF), other familial temporal lobe epilepsies (FTLE) (such as, for example, mesial form of FTLE, familial mesial temporal lobe epilepsy (FMTLE) or familial lateral temporal lobe epilepsy (FLTLE)), familial focal epilepsy with variable foci (FFEVF, childhood to adult), familial partial epilepsy with variable foci (FPEVF), benign familial partial epilepsies of childhood, reflex epilepsies, mesial temporal lobe epilepsy with hippocampal sclerosis (MTLE with HS), temporal lobe epilepsy, idiopathic generalized epilepsy (IGE), Rasmussen syndrome, gelastic seizures with hypothalamic hamartoma, hemiconvulsion-hemiplegia-epilepsy, neurocutaneous syndromes (tuberous sclerosis complex, Sturge-Weber and the like), epilepsies attributed to malformations of cortical development, tumor, infection or trauma, benign neonatal seizures (BNS), febrile seizures (FS) and generalized epilepsy with febrile seizures plus (GEFS+).

In one embodiment, the epileptic disease, disorder or condition is selected from the group comprising autosomal-dominant nocturnal frontal lobe epilepsy (ADNFLE), familial temporal lobe epilepsy (FTLE) and familial focal epilepsy with variable foci (FFEVF).

### DEFINITIONS

In the present invention, the following terms have the following meanings:
- **"Epileptic disease, disorder or condition"** (which may also be referred as an "epileptic syndrome") refers to a disease, disorder or condition characterized by the occurrence of epileptic seizures, preferably of spontaneous and/or recurrent seizures. An epileptic seizure may be defined as an episodic change in the behavior caused by the disordered firing of populations of neurons of the central nervous system, resulting in varying degrees of involuntary muscle contraction, of abnormal perceptions or behaviours, and often an alteration or a loss of consciousness.
- ***"DEPDC5"*** (also known as *KIAA0645*) refers to a human gene on chromosome 22 having the following accession number: NC_000022.10. Five transcript variants of the *DEPDC5* gene were identified, encoding five putative isoforms of *DEPDC5* of length 1572 (NM_014662.3), 559 (NM_001007188.2), 1594 (NM_001136029.2), 1603 (NM_001242896.1) and 1503 (NM_001242897.1) amino acids. In the present patent application, all reference to a position within the *DEPDC5* transcript or within the DEPDC5 protein refers to the transcript encoding the 1603 amino acid protein.
- **"Mutation":** refers to a stable change in the genomic sequence. Examples of mutation include, but are not limited to, point mutations, insertions, inversions, deletions within exons or flanking sequences, or gene rearrangements such as deletions, inversion or duplications of exons.
- A **"Substantially healthy subject"** refers to a subject who has not been previously diagnosed or identified as having or suffering from an epileptic disease, disorder or condition.
- **"About"** preceding a figure means plus or less 10% of the value of said figure

### DETAILED DESCRIPTION

The present invention thus relates to a non-invasive method for diagnosing an epileptic disease, disorder or condition in a subject, or for identifying a subject predisposed to an epileptic disease, disorder or condition, wherein said method comprises determining the status of the *DEPDC5* gene of the subject.

The present invention also relates to a method for classifying a subject as having an epileptic disease, disorder or condition or a predisposition of developing an epileptic disease, disorder or condition or as being at risk of developing an epileptic disease, disorder or condition, wherein said method comprises determining the status of the *DEPDC5* gene of the subject.

The present invention also relates to a method for determining the risk of a subject of developing an epileptic disease, disorder or condition, or for determining the risk of an epileptic disease, disorder or condition onset, wherein said method comprises determining the status of the *DEPDC5* gene of the subject. In one embodiment, said subject presents a familial history of an epileptic disease, disorder or condition or belongs to a kindred wherein at least one member carries a *DEPDC5* mutation.

In one embodiment, determining the status of the *DEPDC5* gene corresponds to detecting the presence of mutations within the *DEPDC5* gene sequence.

In one embodiment of the invention, the presence of at least one mutation within the *DEPDC5* gene of the subject is indicative of the affection of the subject with an epileptic disease, disorder or condition; and/or of the presence of an epileptic disease, disorder or condition in the subject; and/or of an increased risk of the subject to develop an epileptic disease, disorder or condition; and/or of a predisposition of the subject with an epileptic disease, disorder or condition.

In one embodiment, said mutation is a homozygote mutation. In another embodiment, said mutation is a heterozygote mutation.

In one embodiment of the invention, determining the status of the *DEPDC5* gene corresponds to detecting the presence of at least one frameshift mutation within the *DEPDC5* gene sequence, preferably at least one frameshift mutation introducing a premature stop codon within the sequence of the *DEPDC5* gene. Examples of frameshift mutations include, but are not limited to, a mutation deleting an Adenine in position 32200188 of chromosome 22, leading to the deletion of an Adenine in position 1122 of the *DEPDC5* transcript, leading, in the protein, to a premature stop codon 29 amino acids further (mutation Leu374fs*30).

In one embodiment of the invention, determining the status of the *DEPDC5* gene corresponds to detecting the presence of at least one nonsense mutation within the *DEPDC5* gene sequence, leading to the introduction of a codon stop within the sequence of the *DEPDC5* gene. Examples of nonsense mutations include, but are not limited to, mutations presented in the Table 1 below.

**Table 1**

| Mutation in *DEPDC5* gene (position on chromosome 22) | Mutation in *DEPDC5* transcript | Mutation in DEPDC5 protein |
|---|---|---|
| 32188751 C → T | 715 C → T | Arg239* |
| 32198725 C → T | 982 C → T | Arg328* |
| 32200180 C → T | 1114 C → T | Gln372* |
| 32210991 C → T | 1459 C → T | p.Arg487* |
| 32253534 C → T | 3259 C → T | p.Arg1087* |
| 32302238 C → T | 4567 C → T | Gln1523* |

In one embodiment of the invention, determining the status of the *DEPDC5* gene corresponds to detecting the presence of at least one missense mutation within the *DEPDC5* gene sequence. Examples of missense mutations include, but are not limited to, a mutation replacing Guanine by Adenine in position 32210986 of chromosome 22, leading to the replacement of the Guanine in position 1454 by a Adenine in the *DEPDC5* transcript, leading, in the protein, to the replacement of the Arginin of position 485 by a Glycin.

In one embodiment of the invention, determining the status of the *DEPDC5* gene corresponds to detecting the presence of at least one mutation in the *DEPDC5* gene. Examples of mutations include, but are not limited to, the mutations presented in the **Table 2** below.

**Table 2**

| Mutation in *DEPDC5* gene (position on chromosome 22) | Mutation in *DEPDC5* transcript | Mutation in DEPDC5 protein |
|---|---|---|
| 32200188: deletion of A | 1122: deletion of A | Leu374frameshift*30 |
| 32188751 C → T | 715 C → T | Arg239* |
| 32198725 C → T | 982 C → T | Arg328* |
| 32200180 C → T | 1114 C → T | Gln372* |
| 32210991 C → T | 1459 C → T | p.Arg487* |
| 32253534 C → T | 3259 C → T | p.Arg1087* |
| 32302238 C → T | 4567 C → T | Gln1523* |
| 32210986 G → A | 1454 G → A | Arg485Gln |

Methods for detecting mutations within the *DEPDC5* gene sequence are well known from the skilled artisan. Examples of said methods include, but are not limited to, PCR, qPCR, hybridization techniques such as, for example, use of microarrays, Southern Blot or sequencing, such as, for example, exome sequencing, pyrosequencing or Sanger sequencing.

In one embodiment of the invention, the detection of the mutations of *DEPDC5* is performed on a sample from the subject, preferably a body fluid sample from the subject. Examples of body fluids include, but are not limited to, blood, plasma, serum, saliva, lymph, ascetic fluid, cystic fluid, urine, bile, nipple exudate, synovial fluid, bronchoalveolar lavage fluid, sputum, amniotic fluid, chorionic villi, peritoneal fluid, cerebrospinal fluid, pleural fluid, pericardial fluid, semen, saliva, sweat and alveolar macrophages. Preferably, said body fluid is plasma or serum.

Examples of primers that may be used for sequencing the *DEDPC5* gene sequence include, but are not limited to, the pairs of primers of **Table 3** below:

| | **Primer** | **Sequence** | **SEQ ID** |
|---|---|---|---|
| **1** | forward primer | GGGAGGCAAGATGACTTCTC | SEQ ID NO : 1 |
| | reverse primer | TCACCCAGAAGGTGGAGTTT | SEQ ID NO : 2 |
| **2** | forward primer | GGGATATGTGGCGACTAAAG | SEQ ID NO: 3 |
| | reverse primer | CAAAGTGGTGGTGCAGAATG | SEQ ID NO : 4 |
| **3** | forward primer | GAGCCTCAATTCGACAGGAT | SEQ ID NO : 5 |
| | reverse primer | CTCGTCTGGGCGATGACTAT | SEQ ID NO : 6 |
| **4** | forward primer | CGTTACATGCTGTCCCATTG | SEQ ID NO: 7 |
| | reverse primer | ACTGAGGAAGTGAGGAGCCA | SEQ ID NO : 8 |
| **5** | forward primer | CCAGCTCCTGTGATGTTTCA | SEQ ID NO : 9 |
| | reverse primer | TCCAACTCACTCCTCTGCAG | SEQ ID NO : 10 |
| **6** | forward primer | CCAGACCCGACAGAATATGG | SEQ ID NO : 11 |
| | reverse primer | GATGACAGCCCAGCAGGTAT | SEQ ID NO : 12 |
| **7** | forward primer | CAGAAGCAGACATCGACAGG | SEQ ID NO : 13 |
| | reverse primer | GAACGGCTGGAGGAGTACAA | SEQ ID NO : 14 |
| **8** | forward primer | CCTCTGTCCTTCCCACTCAG | SEQ ID NO : 15 |
| | reverse primer | GGAAGAAGGGAACCTCAGCT | SEQ ID NO : 16 |
| **9** | forward primer | CACGCATTCTCTGGAGTCAA | SEQ ID NO : 17 |
| | reverse primer | GCAAGCTCCTTGACCTCATC | SEQ ID NO : 18 |
| **10** | forward primer | CAGGGCTACTCCTCCACAAA | SEQ ID NO: 19 |
| | reverse primer | ACAGCAGGAGTGGACGACTT | SEQ ID NO : 20 |
| **11** | forward primer | ACAAAGAGCCCGACCGAGT | SEQ ID NO : 21 |
| | reverse primer | CTCTTCGAGATGGTCCAAGG | SEQ ID NO : 22 |
| **12** | forward primer | ATCAAGCTGCACTGGATGG | SEQ ID NO : 23 |
| | reverse primer | CCTACTCCAAGCGCAAGTTC | SEQ ID NO : 24 |
| **13** | forward primer | CTTCCCTGCTGAGAACAAGC | SEQ ID NO : 25 |
| | reverse primer | AGGATTCCAGGCAGGCTCTA | SEQ ID NO : 26 |

In one embodiment of the invention, the method of the invention comprises assessing the expression level of *DEPDC5* in the subject. Indeed, the Inventors have shown (see Examples), that *DEPDC5* haploinsufficiency may cause an epileptic disease, disorder or condition in a subject. In one embodiment, the expression level of *DEPDC5* in the subject is compared with the expression level of *DEPDC5* in a reference sample.

In one embodiment of the invention, determining the status of the *DEPDC5* gene thus corresponds to assessing the expression level of *DEPDC5,* preferably detecting *DEPDC5* haploinsufficiency in the subject. In one embodiment, a haploinsufficiency of DEPDC5 may be the consequence of a missense mutation or of a frameshift mutation introducing a premature stop codon within the *DEPDC5* sequence. In another embodiment, a haploinsufficiency of *DEPDC5* may be the consequence of a mutation within the *DEPDC5* gene, wherein the mutated DEPDC5 protein resulting from the expression of the mutated *DEPDC5* gene is addressed to a protein degradation system, such as, for example, proteasome and the like. In another embodiment, a haploinsufficiency of *DEPDC5* may be the consequence of a mutation within the *DEPDC5* gene, wherein the mutated DEPDC5 transcript resulting from the transcription of the mutated *DEPDC5* gene is addressed to a transcript degradation system, such as, for example, the nonsense mediated decay (NMD).

In one embodiment of the invention, the detection of *DEPDC5* haploinsufficiency is performed at the protein level. In another embodiment of the invention, the detection of *DEPDC5* haploinsufficiency is performed at the RNA level.

In one embodiment of the invention, the detection of *DEPDC5* haploinsufficiency comprises measuring the expression level of *DEPDC5* (either at the protein or at the RNA level), and comparing said expression level of *DEPDC5* with a reference expression value.

In one embodiment of the invention, the method of the invention comprises measuring the protein level of the *DEPDC5* protein in a sample from the subject, and comparing said protein level with a reference protein level value.

In one embodiment, said sample from the subject is a body fluid sample from the subject. Examples of body fluids include, but are not limited to, blood, plasma, serum, saliva, lymph, ascetic fluid, cystic fluid, urine, bile, nipple exudate, synovial fluid, bronchoalveolar lavage fluid, sputum, amniotic fluid, chorionic villi, peritoneal fluid, cerebrospinal fluid, pleural fluid, pericardial fluid, semen, saliva, sweat and alveolar macrophages. Preferably, said body fluid is plasma or serum.

Methods for determining a protein level in a body fluid are well-known in the art. Examples of such methods include, but are not limited to, Multiplex methods (Luminex), western blot, enzyme-linked immunosorbent assay (ELISA), sandwich ELISA, fluorescent-linked immunosorbent assay (FLISA), enzyme immunoassay (EIA), radioimmunoassay (RIA) and the like. Examples of antibody anti-DEPDC5 are DEPDC5 (D-19): sc-86116 and DEPDC5 (C-12): sc-86115 (Santa Cruz Biotechnolgy Inc) and DEPD5 Antibody (C-term) (Abgent).

In one embodiment of the invention, the method of the invention comprises measuring the RNA level of the *DEPDC5* transcript in a sample from the subject, and comparing said RNA level with a reference RNA level value.

In one embodiment, said sample from the subject is a body fluid sample from the subject. Examples of body fluids include, but are not limited to, blood, plasma, serum, saliva, lymph, ascetic fluid, cystic fluid, urine, bile, nipple exudate, synovial fluid, bronchoalveolar lavage fluid, sputum, amniotic fluid, chorionic villi, peritoneal fluid, cerebrospinal fluid, pleural fluid, pericardial fluid, semen, saliva, sweat and alveolar macrophages. Preferably, said body fluid is plasma or serum.

Methods for determining a RNA level in a body fluid are well-known in the art. Examples of such methods include, but are not limited to, RT-PCR, RT-qPCR (wherein qPCR stands for quantitative PCR), Northern Blot, hybridization techniques such as, for example, use of microarrays, sequencing such as, for example, next-generation DNA sequencing (NGS) or RNA-seq (also known as "Whole Transcriptome Shotgun Sequencing") and the like.

Examples of PCR or qPCR primers that may be used for assessing the *DEPDC5* RNA expression include, but are not limited to, the pairs of primers of the Table 4 below.

**Table 4**

| | **Primer** | **Sequence** | **SEQ ID** |
|---|---|---|---|
| **14** | forward primer | TCTGACATTCCAACCTTTTCG | SEQ ID NO : 27 |
| | reverse primer | ACACCAGAACCTCAGCCAAC | SEQ ID NO : 28 |
| **15** | forward primer | CTGATGGGGTTGTGAGGAAC | SEQ ID NO : 29 |
| | reverse primer | GCCTTGGACATCATTTGGAA | SEQ ID NO : 30 |
| **16** | forward primer | ACAGAATGGCCAGAGTGACC | SEQ ID NO : 31 |
| | reverse primer | TTACTTGCCCTCATCCCTTG | SEQ ID NO : 32 |
| **17** | forward primer | CGTGTCAGATGATCAATTGTGTT | SEQ ID NO : 33 |
| | reverse primer | AGGAAGGGTGCTTATTCCAAA | SEQ ID NO : 34 |
| **18** | forward primer | GTGTGGCAAAGTAATGTCAGAGA | SEQ ID NO : 35 |
| | reverse primer | GGAAGAACACAGAAGACGTTGA | SEQ ID NO : 36 |
| **19** | forward primer | TCCCTCCCTCCCTCTCTCTA | SEQ ID NO : 37 |
| | reverse primer | GACATCCATACGCCTCCACT | SEQ ID NO : 38 |
| **20** | forward primer | GCTATTGCACCAGGCATGTA | SEQ ID NO : 39 |
| | reverse primer | CAGGTGGGTCTCTCCATGTT | SEQ ID NO : 40 |
| **21** | forward primer | TGAAACTGTATATGGCATTGCTT | SEQ ID NO : 41 |
| | reverse primer | GCATTTGCAGTTCCAGAAAA | SEQ ID NO : 42 |
| **22** | forward primer | CGTTCTACGTCGGCTATGGT | SEQ ID NO : 43 |
| | reverse primer | CGAGAAAACACTCTAACCTCCAA | SEQ ID NO : 44 |
| **23** | forward primer | TGCATGCAGTCTGCTTCATAG | SEQ ID NO: 45 |
| | reverse primer | GGAAAAGTGCAACGAGGAAA | SEQ ID NO : 46 |
| **24** | forward primer | CTTCTTTTTGGTCCCCTTTCTT | SEQ ID NO : 47 |
| | reverse primer | CAGTGGCAAAAGCAAGCATA | SEQ ID NO : 48 |
| **25** | forward primer | AGGGGACACAGAGCCAAAC | SEQ ID NO : 49 |
| | reverse primer | CCCAGACCCCTCCTACACTT | SEQ ID NO : 50 |
| **26** | forward primer | AGATTGTGCTGCTCCACTCC | SEQ ID NO : 51 |
| | reverse primer | TAGGCAAGATGCTGGCTCAT | SEQ ID NO : 52 |
| **27** | forward primer | TGTGACAGGGTTCTGAAAAGC | SEQ ID NO : 53 |
| | reverse primer | AGGGGAAATCAGGCTAGGAA | SEQ ID NO : 54 |
| **28** | forward primer | CCCTCCCCACAATTCTTTTT | SEQ ID NO : 55 |
| | reverse primer | AGCGCCTGGAATTTTTCTCT | SEQ ID NO : 56 |
| **29** | forward primer | TGCTCTCATTTTTCTCCTTGC | SEQ ID NO : 57 |
| | reverse primer | GAAAAATGGCAAGGAAGCTG | SEQ ID NO : 58 |
| **30** | forward primer | GGGCTGATCCATGCCTAGT | SEQ ID NO : 59 |
| | reverse primer | GGCTGAAACAGGAGGACTGA | SEQ ID NO : 60 |
| **31** | forward primer | GCTCCTGCCTTTCTGTCAGT | SEQ ID NO : 61 |
| | reverse primer | GCTGGGATTATAGGCGTGAG | SEQ ID NO : 62 |
| **32** | forward primer | TCAGCTCTCAGGCATGTGTT | SEQ ID NO : 63 |
| | reverse primer | TATATGCACCCCCAAGTGAA | SEQ ID NO : 64 |
| **33** | forward primer | TCCCTCGCTTGATGGTAACT | SEQ ID NO : 65 |
| | reverse primer | ATGTCCCTGGCTCTGTCTGT | SEQ ID NO : 66 |
| **34** | forward primer | GCTCTGGTTTCACTGTGGTTC | SEQ ID NO : 67 |
| | reverse primer | CAAGGCTCTCTCTTAAGCAACC | SEQ ID NO : 68 |
| **35** | forward primer | GGGAATGATGCTCAGTGGTT | SEQ ID NO : 69 |
| | reverse primer | CTGGGTGCTGGAGAGTGTTC | SEQ ID NO : 70 |
| **36** | forward primer | CGGGATATAGGTGAGCAGGA | SEQ ID NO : 71 |
| | reverse primer | TTCTGGCCCTTGTGTCATTT | SEQ ID NO : 72 |
| **37** | forward primer | GCTCCAGTTTGCCTTTTTCA | SEQ ID NO : 73 |
| | reverse primer | CTCCCTGTCTACCCCTTTCC | SEQ ID NO : 74 |
| **38** | forward primer | TTTTCCCCCTGTTACGTGAG | SEQ ID NO : 75 |
| | reverse primer | GCCAAGTGCCTTTTTCTCTG | SEQ ID NO : 76 |
| **39** | forward primer | TGAGCAATCATCTGTTGTTTTCA | SEQ ID NO : 77 |
| | reverse primer | TGAAAACCCACTTCCACATACA | SEQ ID NO : 78 |
| **40** | forward primer | GAGCTTCAAACAGATGGAGGA | SEQ ID NO : 79 |
| | reverse primer | GTGGCCATAATTCAGGACAAT | SEQ ID NO : 80 |
| **41** | forward primer | GGGACCTGCCCTTTATTTTG | SEQ ID NO : 81 |
| | reverse primer | ATCCAAAGCCCACTCTCTGA | SEQ ID NO : 82 |
| **42** | forward primer | ACCACCCTGTGTTTTCCTTG | SEQ ID NO : 83 |
| | reverse primer | AGGCCCTGCATCTCACAC | SEQ ID NO : 84 |
| **43** | forward primer | TGATGGCCTTGTCTCCTCTT | SEQ ID NO : 85 |
| | reverse primer | GGGGACAAGTCAGTCTTCCA | SEQ ID NO : 86 |
| **44** | forward primer | TCTGAGCAAGCTGCTGTCAT | SEQ ID NO : 87 |
| | reverse primer | GCGATCTGTTTTACCCTCCA | SEQ ID NO : 88 |
| **45** | forward primer | TGCTTCCTCCTCCTGTGACT | SEQ ID NO : 89 |
| | reverse primer | CTTAGTTGCAGCCCAATTCC | SEQ ID NO : 90 |
| **46** | forward primer | AGTACAGCTTATTTACTGTGTTTTCCT | SEQ ID NO : 91 |
| | reverse primer | AGCTTTGGACTTCAGCCTC | SEQ ID NO : 92 |
| **47** | forward primer | TTGCTTTGCTGACCTGACAC | SEQ ID NO : 93 |
| | reverse primer | ACTTGCCCAAGCTCTCAAAA | SEQ ID NO : 94 |
| **48** | forward primer | TGGGAGTCCCTTCTTTTTCA | SEQ ID NO : 95 |
| | reverse primer | GCCCCTCTTGCTGTCTCA | SEQ ID NO : 96 |
| **49** | forward primer | CCTGTCACTCCCTTGTCTCC | SEQ ID NO : 97 |
| | reverse primer | GAAAAACACTTGCCCCTGTG | SEQ ID NO : 98 |
| **50** | forward primer | TCCTTCTCTCCCCTCCACTT | SEQ ID NO : 99 |
| | reverse primer | ACCTTGTCTGGGTTGAGCAT | SEQ ID NO : 100 |
| **51** | forward primer | CCCTCCTGTGTGCTGACATA | SEQ ID NO : 101 |
| | reverse primer | GAGCCAAAGGCCTGACAA | SEQ ID NO : 102 |
| **52** | forward primer | GGGTTTTCTCCTTGGGAAGTA | SEQ ID NO : 103 |
| | reverse primer | CGGGGTCTTACTGGTTGAGA | SEQ ID NO : 104 |
| **53** | forward primer | ACAGAAGACTGGCCCAGAGA | SEQ ID NO : 105 |
| | reverse primer | TTCCCACTCTTCACACTTGCT | SEQ ID NO : 106 |
| **54** | forward primer | GCATGTCTTCCTGTCCTTCC | SEQ ID NO : 107 |
| | reverse primer | AGGTGGTTCTGCAGCCATAG | SEQ ID NO : 108 |
| **55** | forward primer | CGCTCCCCTTTCTCTTCAG | SEQ ID NO : 109 |
| | reverse primer | CACCTTCCCCCAGCACAG | SEQ ID NO : 110 |
| **56** | forward primer | AGTAGGCGCTTCAGGCTTAGG | SEQ ID NO : 111 |
| | reverse primer | GATTCCAGGCAGGCTCTAGGA | SEQ ID NO : 112 |

In one embodiment, the status of the *DEPDC5* gene of the subject is compared with the status of the *DEPDC5* gene of a reference population.

In one embodiment, the reference value (which may correspond to a reference expression value, a reference protein level value or a reference RNA level value as hereinabove described) corresponds to a value measured in a reference population.

A reference value can be relative to an expression value derived from population studies, including without limitation, such subjects having similar age range, subjects in the same or similar ethnic group, similar epileptic disease, disorder or condition history and the like.

In one embodiment, the reference value is constructed using algorithms and other methods of statistical and structural classification.

In one embodiment of the invention, the reference value is derived from the measurement of the expression value in a control sample derived from one or more substantially healthy subjects.

In another embodiment of the invention, the reference value is derived from the measurement of the expression value of the *DEPDC5* gene in a reference sample, preferably a reference body fluid sample, derived from a reference population.

In one embodiment, the reference population comprises substantially healthy subjects, preferably at least 100, more preferably at least 250, more preferably at least 500 substantially healthy subjects.

In another embodiment, the reference population comprises subjects diagnosed with an epileptic disease, disorder or condition, preferably at least 100, more preferably at least 250, more preferably at least 500 subjects diagnosed with an epileptic disease, disorder or condition.

In another embodiment, the reference population comprises subjects carrying out a mutation of the *DEPDC5* gene, preferably at least 100, more preferably at least 250, more preferably at least 500 subjects carrying out a mutation of the *DEPDC5* gene.

In one embodiment, the reference value corresponds to the mean expression value of the *DEPDC5* gene measured in the reference population. In one embodiment of the invention, the reference value corresponds to the median expression value of the *DEPDC5* gene measured in the reference population.

In one embodiment of the invention, a reduced expression of the *DEPDC5* gene as compared to a reference expression value is indicative of the affection of the subject with an epileptic disease, disorder or condition. In one embodiment of the invention, a reduced expression of the *DEPDC5* gene as compared to a reference expression value is indicative of the presence of an epileptic disease, disorder or condition in the subject. In one embodiment of the invention, a reduced expression of the *DEPDC5* gene as compared to a reference expression value is indicative of an increased risk of the subject to develop an epileptic disease, disorder or condition. In one embodiment of the invention, a reduced expression of the *DEPDC5* gene as compared to a reference expression value is indicative of a predisposition of the subject with an epileptic disease, disorder or condition.

In one embodiment, a reduced expression corresponds to a percent reduction of about 5% compared to the reference value, preferably a percent reduction of about 10%, 15%, 20%, 25%, 30%, 40%, 50% or more. Preferably, the reference expression value was measured in a reference population comprising substantially healthy subjects.

In one embodiment of the invention, a reduced expression of *DEPDC5* is indicative of a *DEPDC5* haploinsufficiency in the subject.

The ILAE (International League Against Epilepsy) has published in 2010 a revised classification of epileptic diseases, disorders or conditions (Berg et al, Epilepsia, 51(4):676-685, which is herein incorporated by reference). According to said classification, epileptic diseases, disorders or conditions may be classified according to the seizure type (generalized seizures, focal seizures, or spasms), etiology (genetic

[including idiopathic], structural/metabolic [or symptomatic], or unknown cause [or cryptogenic]), age at onset, cognitive and developmental antecedents and consequences, motor and sensory examinations, EEG features, provoking or triggering factors, and/or patterns of seizure occurrence with respect to sleep.

Examples of epileptic diseases, disorders or conditions include, but are not limited to, benign familial neonatal epilepsy (BFNE), early myoclonic encephalopathy (EME), Ohtahara syndrome, epilepsy of infancy with migrating focal seizures, West syndrome, Myoclonic epilepsy in infancy (MEI), benign infantile epilepsy, benign familial infantile epilepsy, Dravet syndrome, myoclonic encephalopathy in non-progressive disorders, febrile seizures plus (FS+), Panayiotopoulos syndrome, epilepsy with myoclonic atonic seizures, benign epilepsy with centrotemporal spikes (BECTS), autosomal-dominant nocturnal frontal lobe epilepsy (ADNFLE), late onset childhood occipital epilepsy, epilepsy with myoclonic absences, Lennox-Gastaut syndrome, epileptic encephalopathy with continuous spike-and-wave during sleep (CSWS), Landau-Kleffner syndrome (LKS), childhood absence epilepsy (CAE), juvenile absence epilepsy (JAE), juvenile myoclonic epilepsy (JME), epilepsy with generalized tonic-clonic seizures alone, progressive myoclonus epilepsies (PME), autosomal dominant epilepsy with auditory features (ADEAF), other familial temporal lobe epilepsies (FTLE) (such as, for example, mesial form of FTLE, familial mesial temporal lobe epilepsy (FMTLE) or familial lateral temporal lobe epilepsy (FLTLE)), familial focal epilepsy with variable foci (FFEVF, childhood to adult), familial partial epilepsy with variable foci (FPEVF), benign familial partial epilepsies of childhood, reflex epilepsies, mesial temporal lobe epilepsy with hippocampal sclerosis (MTLE with HS), temporal lobe epilepsy, idiopathic generalized epilepsy (IGE), Rasmussen syndrome, gelastic seizures with hypothalamic hamartoma, hemiconvulsion-hemiplegia-epilepsy, neurocutaneous syndromes (tuberous sclerosis complex, Sturge-Weber and the like), epilepsies attributed to malformations of cortical development, tumor, infection or trauma, benign neonatal seizures (BNS), febrile seizures (FS) and generalized epilepsy with febrile seizures plus (GEFS+).

In one embodiment of the invention, the epileptic disease, disorder or condition is a focal epilepsy.

In one embodiment, the epileptic disease, disorder or condition is a genetic or familial epilepsy.

In one embodiment, the epileptic disease, disorder or condition is a familial focal epilepsy. Examples of familial focal epilepsies include, but are not limited to, autosomal-dominant nocturnal frontal lobe epilepsy (ADNFLE), familial temporal lobe epilepsy (FTLE), familial mesial temporal lobe epilepsy (FMTLE), familial lateral temporal lobe epilepsy (FLTLE), familial partial epilepsy with variable foci (FPEVF) and benign familial partial epilepsies of childhood. Preferably, the epileptic disease, disorder or condition is selected from the group comprising autosomal-dominant nocturnal frontal lobe epilepsy (ADNFLE), familial temporal lobe epilepsy (FTLE) and familial focal epilepsy with variable foci (FFEVF).

In one embodiment, the subject is an animal, preferably a mammal, more preferably a human.

In one embodiment, the subject presents a familial history of an epileptic disease, disorder or condition.

In another embodiment, the subject does not present any familial history of an epileptic disease, disorder or condition.

In one embodiment, the subject is substantially healthy.

In one embodiment, the subject is diagnosed with an epileptic disease, disorder or condition.

In one embodiment, the subject is at risk of developing an epileptic disease, disorder or condition. Examples of risk factors for developing an epileptic disease, disorder or condition include, but are not limited to, familial history of epileptic diseases, disorders or conditions, or belonging to a kindred wherein at least one member carries a mutation of the *DEPDC5* gene.

In one embodiment, the assessment of the status of the *DEPDC5* gene of a subject is performed before the onset of the epileptic disease, disorder or condition or of symptoms thereof. In another embodiment, the assessment of the status of the *DEPDC5* gene of a subject is performed after the onset of the epileptic disease, disorder or condition or of symptoms thereof.

In one embodiment of the invention, an onset of an epileptic disease, disorder or condition has been diagnosed in the subject. In another embodiment, no onset of an epileptic disease, disorder or condition has been diagnosed in the subject.

In one embodiment of the invention, the method further comprises performing clinical tests for diagnosing an epileptic disease, disorder or condition in the subject. In one embodiment, the clinical tests are performed previously, simultaneously or following the assessment of the status of the *DEPDC5* gene of the subject.

Examples of clinical tests that may be used for diagnosing an epileptic disease, disorder or condition in a subject include, but are not limited to, electroencephalogram (EEG), video-EEG of the seizures, neuroimaging methods, sleep tests, and the like.

### BRIEF DESCRIPTION OF THE DRAWINGS

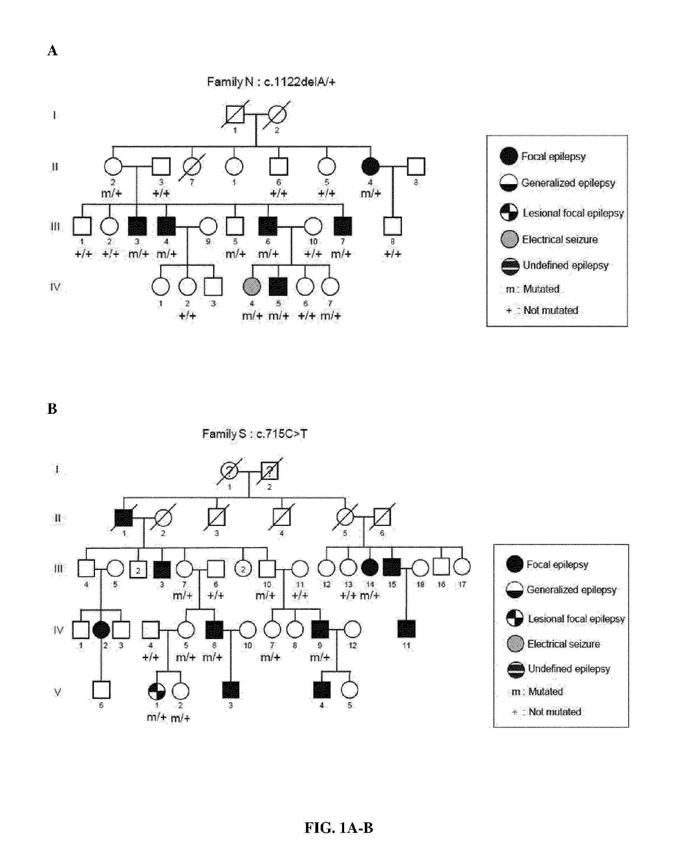

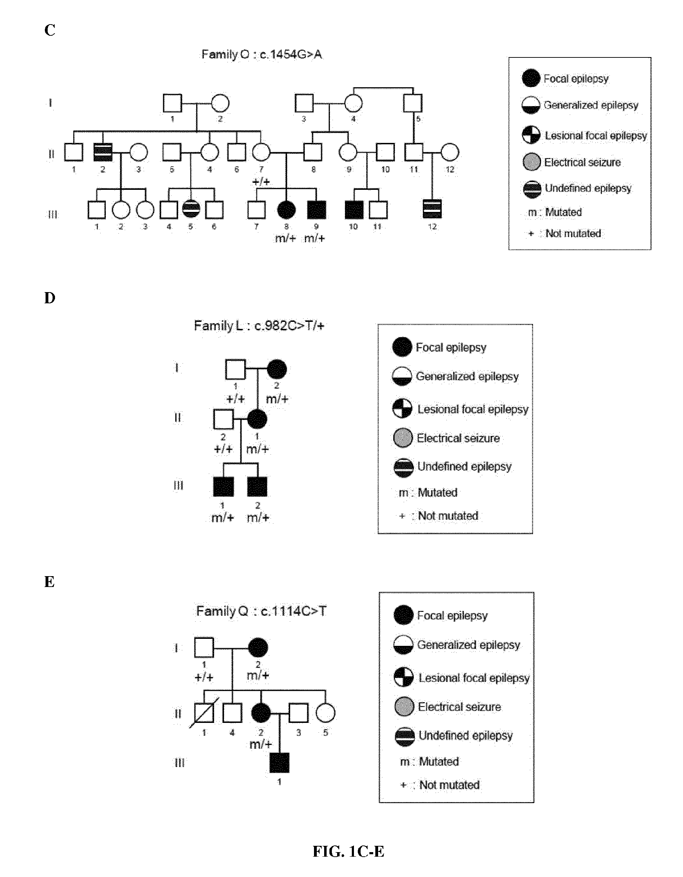

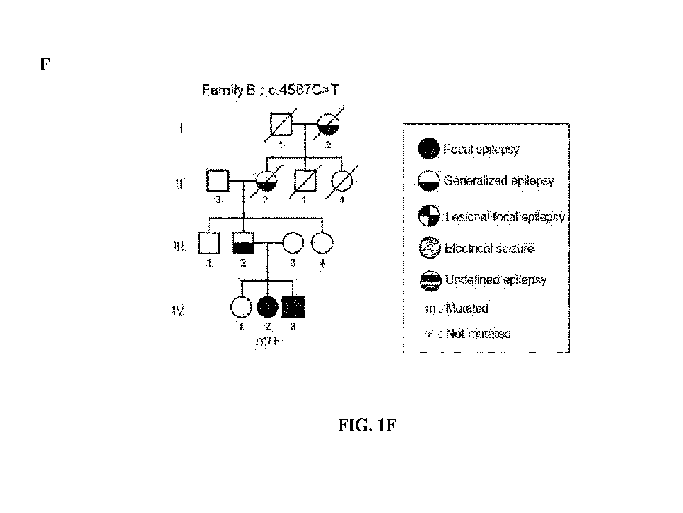

**Figure 1** is a combination of pedigrees of families with segregation of *DEPDC5* mutations: c.1122delA (p.Leu374fs*30) in family N (**A**), c.715C>T (p.Arg239*) in family S (**B**), c.1454G>A (p.Arg485Gln) in family O (**C**), c.982C>T (p.Arg328*) in family L (**D**), c.1114C>T (p.Gln372*) in family Q (**E**), c.4567C>T (p.Gln1523*) in family B (**F**). Individual N-IV:4 showed frontal spikes at the EEG, but no clinical seizure; indicates individuals born in the 1870s, with doubtful epilepsy histories. Diagonal lines indicate deceased individuals.

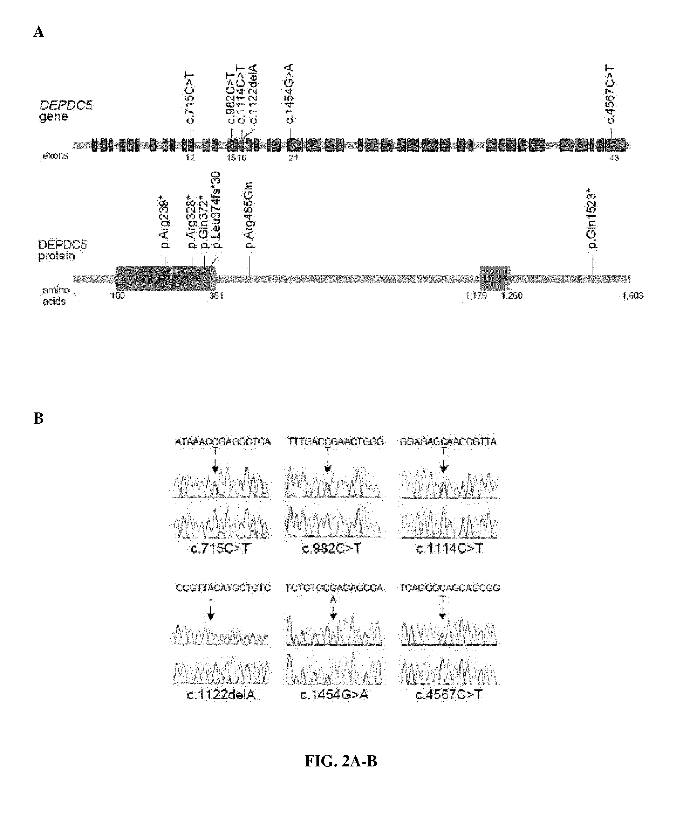

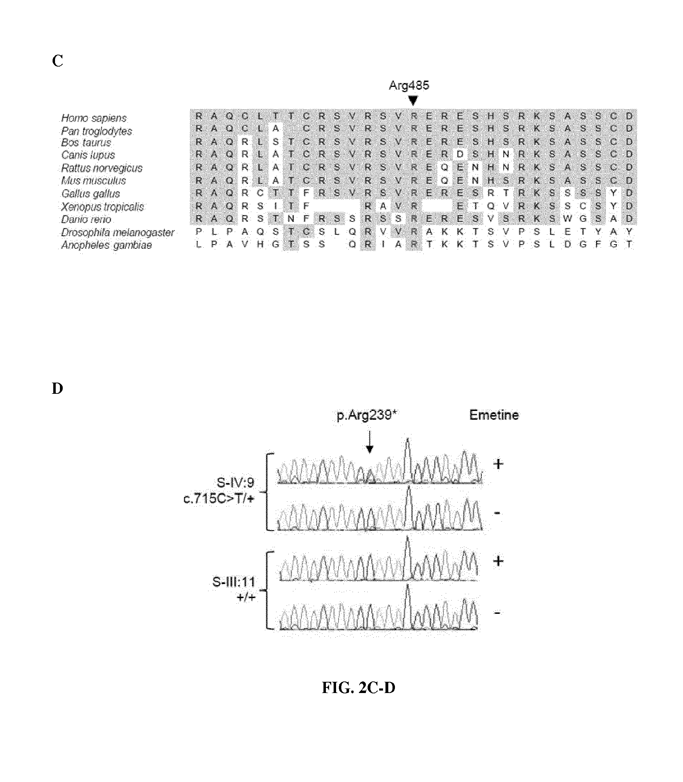

**Figure 2** is a combination of illustrations and sequence chromatogram. (**A**) Illustration of the exon-intron structure of *DEPDC5* gene with the position of the mutations based on the human genomic sequence (RefSeq NM_001242896) and protein. DEPDC5 is a multidomain protein carrying an N-terminal domain of unknown function termed DUF3608 and a DEP domain at the C-terminal. Mutations p.Arg239*, p.Arg328*, p.Gln372*, p.Leu374fs*30 are clustered in the DUF3608 domain. (**B**) Sequence chromatograms of all *DEPDC5* mutations. (**C**) Multiple protein alignment showing conservation of the arginine residue in position 485 in orthologs of DEPDC5 across species. (**D**) Sequence chromatograms of one affected (S-IV:9) and one unaffected (S-III:11) member of family S, showing the presence of p.Arg239* mutation in *DEPDC5* cDNA of S-IV:9 lymphoblasts pretreated with emetine, but not in untreated cells.

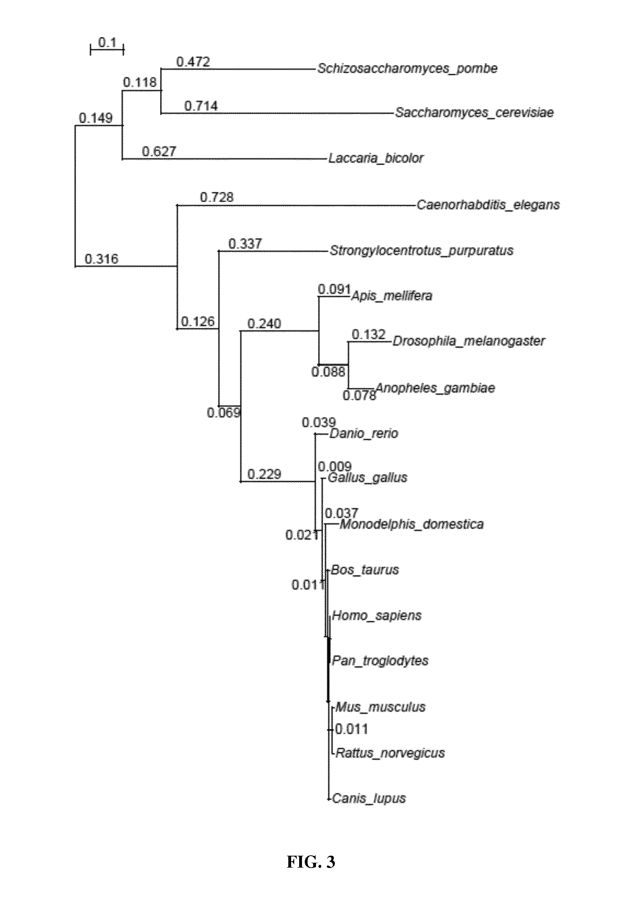

**Figure 3** is a phylogenic tree revealing a highly conserved DEPDC5 protein present in metazoa and fungi.

### EXAMPLES

The present invention is further illustrated by the following examples.

### Methods

### Families and patients

Sixteen unrelated families from Western Europe with non-lesional focal epilepsies were studied (Picard, F. et al. Brain 123 (6), 1247-62 (2000)). Mutations in *CHRNA4, CHRNB2* and *LGI1* have been excluded in all families. Local ethics committee (CCPPRB of Pitié-Salpêtrière Hospital, Paris, No. 69-03, 25/9/2003) approved this study. Informed consent was obtained from all participants or parents.

### Genotyping and linkage analysis

A genome-wide screen was performed on family N and S with Affymetrix 10K panel microarrays (IntegraGen, Evry, France). LOD scores were calculated with MERLIN software assuming an autosomal dominant trait with 70% penetrance, a disease frequency of 0.0001 and 0% phenocopy. Haplotypes were reconstructed according to hg19 annotations.

### Exome sequencing

Exome sequencing was carried out in patients III-4 and III-6 of family N. Targeted exome sequencing, library preparation, capture, sequencing, and variant detection and annotation, were performed by IntegraGen (Evry, France). Exons of genomic DNA samples were captured using Agilent in-solution enrichment methodology with a biotinylated oligonucleotide probes library, followed by paired-end 75 bases massively parallel sequencing on Illumina HiSEQ2000. Sequence capture was performed according to the manufacturer's instructions (Human All exon kit V4+UTRs, 70 Mb, Agilent). Briefly, 3 µg of each genomic DNA were fragmented by sonication and purified to yield fragments of 150-200 bp. Paired-end adaptor oligonucleotides from Illumina were ligated on repaired A-tailed fragments, then purified and enriched by 6 PCR cycles. Purified libraries (500 ng) were hybridized to the SureSelect oligo probe capture library for 24 hr. After hybridization, washing, and elution, the eluted fraction was PCR-amplified with 10 to 12 cycles, purified and quantified by qPCR to obtain sufficient DNA template for downstream applications. Each eluted-enriched DNA sample was then sequenced on an IlluminaHiSEQ2000 as paired-end 75 bases reads. Image analysis and base calling were performed using Illumina Real Time Analysis Pipeline version 1.14 with default parameters.

### Bioinformatics analysis

Bioinformatics analysis of sequencing data was based on the Illumina pipeline (CASAVA 1.8). CASAVA aligns reads to the human reference genome (hg19) with the alignment algorithm ELANDv2 (performs multiseed and gapped alignments), then calls the SNPs based on the allele calls and read depth, and detects variants (SNPs & Indels). Only positions included in the bait coordinates were conserved. Genetic variation was annotated with the IntegraGen in-house pipeline, consisting of gene annotation (RefSeq), detection of known polymorphisms (dbSNP135, 1000 Genomes Project database) and characterization of mutations as exonic, intronic, silent or nonsense.

### Screening of a cohort by pyrosequencing

All 43 exons and intron-exon junctions of *DEPDC5* (except exon 2 screened by Sanger sequencing) were analyzed by Universal tailed amplicon sequencing (454 Sequencing Technology, Roche). This approach used the 454 GS Junior system and involved an initial PCR amplification with primer sets designed to amplify exons of accession number NM_001242896, a second PCR aiming to incorporate multiplex identifier and 454 adaptors and finally an emulsion PCR step done according to the emPCR Amplification Method Manual.

### Validation of exome and pyrosequencing variants by Sanger sequencing

Mutations found with exome and pyrosequencing were validated by Sanger sequencing using the Big-Dye® Terminator kit on an ABI Prism 3730 DNA Analyzer (Applied Biosystems). Segregation analysis of within-family variants was done using the same primers as for pyrosequencing. Mutations interpretation was assessed with Alamut version 2.2 (Interactive Biosoftware, France). The effects of amino acid substitutions on protein function were predicted using Mutation Taster, SIFT and Polyphen2.

### Cell culture and mRNA experiments

Lymphoblastic cells from three mutation carriers (S-III:10, S-IV:7, S-IV:9) and one spouse (S-III:11) from Family S were treated (or not) overnight with 10 mg/ml emetine to inhibit nonsense-mediated decay (NMD). Total RNA was extracted with the QIAGEN RNeasy Mini kit and reverse-transcribed with the ThermoScript™ RT-PCR System (Invitrogen). *DEPDC5* cDNA was amplified and sequenced using specific primers located in exons 12 and 15.

### Phylogenetic tree

All amino acid sequences of orthologous genes were retrieved from NCBI and aligned using MUSCLE. Visual inspection of the alignment with SEAVIEW shows that a homology dispersed across several regions of the protein. Protein alignment was trimmed to 454 homologous sites using GBLOCK. The tree reconstruction was performed by maximum likelihood using the software PHYML with an LG matrix.

### Results

We have previously described the electro-clinical characteristics of 19 families with autosomal dominant focal epilepsies, subdivided into ADNFLE, FTLE and FFEVF (Picard, F. et al. Brain 123 (6), 1247-62 (2000)). Pedigrees of 6 of these families are shown in **Figure 1****.**

Two large multiplex French families (N and S) with diagnosed FFEVF were selected for linkage analysis using a high-density genome-wide scan with 10,000 SNPs. Both families mapped to the FFEVF locus on chromosome 22q12, with a maximum LOD score of 2.08 (family N) and 1.81 (family S). Haplotype reconstruction confirmed the segregation of a disease haplotype in each family.

We then sequenced the entire exome of patients N-III:4 and N-III:6 and sought rare coding variants in the 22q12 candidate region. In both patients we found the same single base deletion, c.1122de1A, in exon 16 of the *DEPDC5* (also known as *KIAA0645*) gene. This variant caused a frameshift, p.Leu374fs*30, introducing a premature stop codon 29 amino acids further. The c.1122de1A variant fully segregated within the family (**Figure 1A**).

We next sought *DEPDC5* mutations in 15 additional probands of these families with autosomal dominant focal epilepsies (ADNFLE= 7, FTLE= 4, FFEVF= 4; Picard et al, 2000), including family S linked to the 22q12 locus. Massively parallel pyrosequencing screening of all 43 coding exons and splice regions of *DEPDC5* revealed four further nonsense mutations (p.Arg239*/family S, p.Arg328*/family L, p.Gln372*/family Q, p.Gln1523*/family B) and one missense mutation (p.Arg485Gln/family O) in 5 families (**Figures 2A-B****, Table 5**).

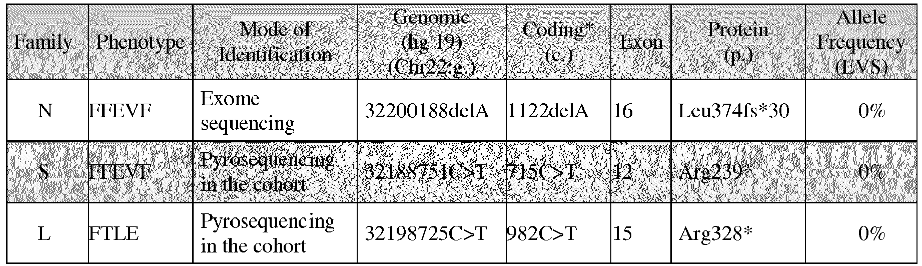

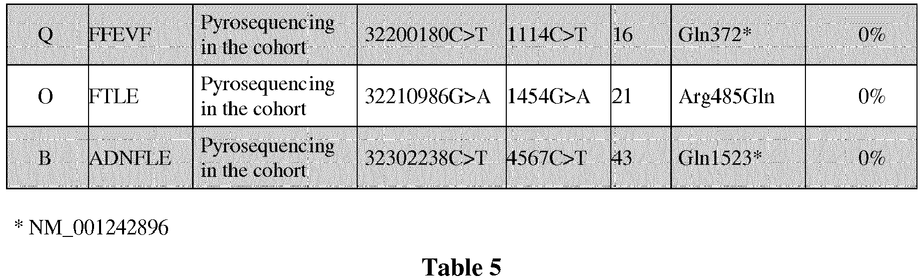

All mutations were shown to segregate within the families, except for p.Gln1523* in family B, where additional family members were unavailable for analysis (**Figure 1**). In family O, the father (O-II:8) may have transmitted the p.Arg485Gln mutation since it was not inherited from the mother (O-II:7). The arginine at position 485 is a highly conserved amino acid (**Figure 2C**). Different prediction software tools - Mutation Taster, SIFT and Polyphen2 - predict that the arginine to glutamine at position 485 is disease causing, tolerated and possibly damaging, respectively. This substitution, p.Arg485Gln, was not detected in a cohort of 450 ethnically matched controls. None of the six *DEPDC5* variants was present in the dbSNP135, 1000 Genomes Project database or the 6,503 exomes for which variants are currently listed in the NHLBI exome variant server (EVS) database. One nonsense (1/11,813) and one frameshift (1/11,775) mutation have been identified in the 6,503 exomes listed in the EVS database and no copy number variations disrupting the *DEPDC5* coding sequence have been reported in the database of genomic variants. These mutations occurred significantly more often in our group of patients (5/32; P = 5.10⁻¹² Fisher exact test).

Four out of six mutations (p.Arg239*, p.Arg328*, p.Gln372*, p.Leu374fs*30) are predicted to be degraded by the nonsense mediated decay (NMD) system, while the nonsense mutation p.Gln1523*, located in the last exon of the gene, is not likely to be targeted by the NMD system. To confirm that the p.Arg239* mutation leads to NMD degradation, we treated cultured lymphoblasts from 3 mutation carriers (S-III:10, S-IV:7 and S-IV:9) and one spouse (S-III:11) with emetine, a NMD inhibitor. Sequencing of *DEPDC5* cDNA revealed that the p.Arg239* mutation is only detected when NMD is inhibited (**Figure 2D**), demonstrating that this nonsense mutation specifically leads to transcript degradation. *DEPDC5* haploinsufficiency is likely to be the cause of the disease.

We identified mutations in one third of the families in our cohort (6/16, 37%) and, overall, 20 patients with epilepsy carried a *DEPDC5* mutation. The age of onset ranged from 0 to 39 years (mean: 12.9 ±10.9 s.d.). *DEPDC5* mutations were not limited to families with familial focal epilepsy with variable foci (FFEVF) phenotype but were also found in a family with two patients exhibiting a typical nocturnal frontal lobe epilepsy (family B) (Thomas, P. et al. Rev Neurol (Paris) 154, 228-35 (1998)) and another family including four patients with temporal lobe seizures (family L). A phenotype of FFEVF was attributed to family N because frontal seizures have been diagnosed in one patient (N-IV:5) and frontal electrical seizures in another (N-IV:4) while seizures in other family members originate in the temporal lobe. Penetrance was incomplete in families N, S and O, in agreement with reports of low penetrance of 62 % (Scheffer, I.E. et al. Ann Neurol 44, 890-9 (1998)) or 50 % (Klein, K.M. et al. Epilepsia 53, e151-5 (2012)) in families with FFEVF. Seven asymptomatic obligate carriers (N-II:2, S-II:5, S-III:4, S-III:7, S-III:10, S-IV:5, O-II:8) and four asymptomatic at-risk individuals aged 8-42 (N-111:5, N-IV:7, S-IV:7, S-V:2) carried the mutations. Our data strongly support the causal role of *DEPDC5* in a subset of our families with focal epilepsies. However, the observed reduced penetrance and variable expressivity (age at onset, seizure focus in various brain areas) suggest that other gene(s) or epigenetic and environmental factors modulate the phenotype.

Since five out of the six mutations introduce a premature stop mutation, a loss-of-function of *DEPDC5* presumably causes the epileptic phenotype. It will now be crucial to determine the function of *DEPDC5* and how its loss causes epilepsy. *DEPDC5* transcript was first identified in human brain cDNA libraries (Ishikawa, K. et al. DNA Res 5, 169-76 (1998)). It is strongly expressed at rather constant levels in both developing and adult human brain (Human Brain Transcriptome project), consistent with a role in epilepsy. We attempted to obtain clues from sequence analysis and database mining. The Reference Sequence (RefSeq) collection reports five putative isoforms of *DEPDC5,* of lengths 1572, 559, 1594, 1603 and 1503 amino acids. One of the mutations (p.Gln1523*) lies within an exon absent from isoform 2, suggesting this isoform is not involved in the pathology. Alignment with orthologs, using reciprocal best BLASTP hits, and phylogeny revealed a highly conserved protein present in metazoa and fungi (but absent from plant) (**Figure 3**), indicating a core role for this ancient eukaryotic gene. We note also that *DEPDC5,* and orthologs, occur as single gene copies even within genomes known to be ancient polyploids, such as *D. rerio* and *S. cerevisiae.* This, together with the dominance of epilepsy-causing mutations, suggests that gene dosage may be critical. Transmembrane prediction using Hidden Markov Models (TMHMM) analysis also shows that *DEPDC5* has no transmembrane domain and no homology with known ion channels. The presence of a DEP domain (Dishevelled, Egl-10 and Pleckstrin domain), a globular protein motif of about 80 amino acids present in many proteins of G-protein signaling pathways, suggests this gene may be a G-protein substrate. *DEPDC5* ortholog in yeast *Yjr138p* (also known as *SEA1*) is part of an evolutionary conserved multi-protein SEA complex involved in membrane trafficking (Dokudovskaya, S. et al. Mol Cell Proteomics 10, M110 006478 (2011)). *DEPDC5* may also be involved in oncology: i) it was reported to be deleted in two glioblastomas (Seng, T.J. et al. Genes Chromosomes Cancer 43, 181-93 (2005)), ii) an intronic SNP (rs1012068) in the *DEPDC5* locus has been associated with hepatocellular carcinoma risk (Miki, D. et al. Nat Genet 43, 797-800 (2011)).

In conclusion, this work provides clear evidence that loss-of-function mutations of *DEPDC5* are a major cause in a broad spectrum of autosomal dominant focal epilepsies. It shows that a single gene, *DEPDC5,* is a common genetic actor for epileptic syndromes with different brain localization and electro-clinical expression, including nocturnal frontal lobe epilepsy (ADNFLE), familial temporal lobe epilepsy (FTLE) and familial focal epilepsy with variable foci (FFEVF).

## Claims

1. A method for diagnosing an epileptic disease, disorder or condition in a subject, or determining a predisposition of a subject to an epileptic disease, disorder or condition or determining an increased risk of a subject of developing an epileptic disease, disorder or condition, wherein said method comprises determining the status of the *DEPDC5* gene of the subject.

2. The method according to claim **1,** comprising detecting at least one mutation in the *DEDPC5* gene sequence in a sample from the subject.

3. The method according to claim **2,** wherein the at least one mutation is a frameshift mutation, preferably a frameshift mutation introducing a premature stop codon within the sequence of the *DEPDC5* gene; a missense mutation or a nonsense mutation or a gene rearrangement.

4. The method according to anyone of claims **1** to **3,** comprising detecting at least one mutation listed in Table 2 in a sample from the subject.

5. The method according to claim **1,** comprising assessing the expression level of *DEPDC5* in a sample from the subject, and comparing said expression level with a reference expression value.

6. The method according to claim **5,** wherein the expression level of *DEPDC5* is measured at the protein level.

7. The method according to claim **5,** wherein the expression level of *DEPDC5* is measured at the RNA level.

8. The method according to anyone of claims **5** to **7,** wherein a reduced expression of DEPDC5 in the sample from the subject is indicative of the affection of the subject with an epileptic disease, disorder or condition; and/or of the presence of an epileptic disease, disorder or condition in the subject; and/or of an increased risk of the subject to develop an epileptic disease, disorder or condition; and/or of a predisposition of the subject with an epileptic disease, disorder or condition.

9. The method according to anyone of claims **5** to **8,** wherein the reference expression value is measured in a reference population, preferably a reference population comprising substantially healthy subjects.

10. The method according to anyone of claims **1** to **9,** wherein the sample is a body fluid sample, preferably blood sample.

11. The method according to anyone of claims **1** to **10,** wherein the subject presents a familial history of an epileptic disease, disorder or condition.

12. The method according to anyone of claims **1** to **11,** wherein an onset of an epileptic disease, disorder or condition has been diagnosed in the subject.

13. The method according to anyone of claims **1** to **11,** wherein no onset of an epileptic disease, disorder or condition has been diagnosed in the subject.

14. The method according to anyone of claims **1** to **13,** wherein the epileptic disease, disorder or condition is selected from the list comprising benign familial neonatal epilepsy (BFNE), early myoclonic encephalopathy (EME), Ohtahara syndrome, epilepsy of infancy with migrating focal seizures, West syndrome, Myoclonic epilepsy in infancy (MEI), benign infantile epilepsy, benign familial infantile epilepsy, Dravet syndrome, myoclonic encephalopathy in non-progressive disorders, febrile seizures plus (FS+), Panayiotopoulos syndrome, epilepsy with myoclonic atonic seizures, benign epilepsy with centrotemporal spikes (BECTS), autosomal-dominant nocturnal frontal lobe epilepsy (ADNFLE), late onset childhood occipital epilepsy, epilepsy with myoclonic absences, Lennox-Gastaut syndrome, epileptic encephalopathy with continuous spike-and-wave during sleep (CSWS), Landau-Kleffner syndrome (LKS), childhood absence epilepsy (CAE), juvenile absence epilepsy (JAE), juvenile myoclonic epilepsy (JME), epilepsy with generalized tonic-clonic seizures alone, progressive myoclonus epilepsies (PME), autosomal dominant epilepsy with auditory features (ADEAF), other familial temporal lobe epilepsies (FTLE) (such as, for example, mesial form of FTLE, familial mesial temporal lobe epilepsy (FMTLE) or familial lateral temporal lobe epilepsy (FLTLE)), familial focal epilepsy with variable foci (FFEVF, childhood to adult), familial partial epilepsy with variable foci (FPEVF), benign familial partial epilepsies of childhood, reflex epilepsies, mesial temporal lobe epilepsy with hippocampal sclerosis (MTLE with HS), temporal lobe epilepsy, idiopathic generalized epilepsy (IGE), Rasmussen syndrome, gelastic seizures with hypothalamic hamartoma, hemiconvulsion-hemiplegia-epilepsy, neurocutaneous syndromes (tuberous sclerosis complex, Sturge-Weber and the like), epilepsies attributed to malformations of cortical development, tumor, infection or trauma, benign neonatal seizures (BNS), febrile seizures (FS) and generalized epilepsy with febrile seizures plus (GEFS+).

15. The method according to anyone of claims **1** to **14,** wherein the epileptic disease, disorder or condition is selected from the group comprising autosomal-dominant nocturnal frontal lobe epilepsy (ADNFLE), familial temporal lobe epilepsy (FTLE) and familial focal epilepsy with variable foci (FFEVF).


SEQUENCE LISTING

<110> ICM (INSTITUT DU CERVEAU ET DE LA MOELLE ÉPINIÈRE)
      CENTRE NATIONAL DE LA RECHERCHE SCIENTIFIQUE (CNRS)
      INSTITUT NATIONAL DE LA SANTÉ ET DE LA RECHERCHE MÉDICALE
      (INSERM)
      APHP (Assistance Publique - Hôpitaux de Paris)
      UNIVERSITÉ PIERRE ET MARIE CURIE - PARIS 6 (UPMC)
      BAULAC, Stéphanie et al.

<120> MUTATIONS OF DEPDC5 FOR DIAGNOSING EPILEPSIC DISEASES, DISORDERS OR
CONDITIONS

<130> CV - 261/EP

<160> 112

<170> BiSSAP 1.2

<210> 1
<211> 20
<212> DNA
<213> Artificial Sequence

<220>
<221> source
<222> 1..20
<223> /mol_type="unassigned DNA"
      /note="Forward primer"
      /organism="Artificial Sequence"

<400> 1
gggaggcaag atgacttctc                                                   20


<210> 2
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 2
tcacccagaa ggtggagttt                                                   20


<210> 3
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 3
gggatatgtg gcgactaaag                                                   20


<210> 4
<211> 20
<212> DNA

<213> Artificial Sequence

<220>
<221> source
<222> 1..20
<223> /mol_type="unassigned DNA"
      /note="Reverse Primer"
      /organism="Artificial Sequence"

<400> 4
caaagtggtg gtgcagaatg                                                   20


<210> 5
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 5
gagcctcaat tcgacaggat                                                   20


<210> 6
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 6
ctcgtctggg cgatgactat                                                   20


<210> 7
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 7
cgttacatgc tgtcccattg                                                   20


<210> 8
<211> 20
<212> DNA
<213> Artificial Sequence

<220>
<221> source
<222> 1..20
<223> /mol_type="unassigned DNA"

/note="Reverse Primer"
      /organism="Artificial Sequence"

<400> 8
actgaggaag tgaggagcca                                                   20


<210> 9
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 9
ccagctcctg tgatgtttca                                                   20


<210> 10
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 10
tccaactcac tcctctgcag                                                   20


<210> 11
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 11
ccagacccga cagaatatgg                                                   20


<210> 12
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 12
gatgacagcc cagcaggtat                                                   20

<210> 13
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 13
cagaagcaga catcgacagg                                                   20


<210> 14
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 14
gaacggctgg aggagtacaa                                                   20


<210> 15
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 15
cctctgtcct tcccactcag                                                   20


<210> 16
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 16
ggaagaaggg aacctcagct                                                   20


<210> 17
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 17
cacgcattct ctggagtcaa                                                   20


<210> 18
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 18
gcaagctcct tgacctcatc                                                   20


<210> 19
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 19
cagggctact cctccacaaa                                                   20


<210> 20
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 20
acagcaggag tggacgactt                                                   20


<210> 21
<211> 19
<212> DNA
<213> Artificial Sequence

<220>
<221> source
<222> 1..19
<223> /mol_type="unassigned DNA"
      /note="Forward Primer"
      /organism="Artificial Sequence"

<400> 21
acaaagagcc cgaccgagt                                                    19


<210> 22
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 22
ctcttcgaga tggtccaagg                                                   20


<210> 23
<211> 19
<212> DNA
<213> Artificial Sequence

<220>
<221> source
<222> 1..19
<223> /mol_type="unassigned DNA"
      /note="Forward Primer"
      /organism="Artificial Sequence"

<400> 23
atcaagctgc actggatgg                                                    19


<210> 24
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 24
cctactccaa gcgcaagttc                                                   20


<210> 25
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 25
cttccctgct gagaacaagc                                                   20


<210> 26

<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 26
aggattccag gcaggctcta                                                   20


<210> 27
<211> 21
<212> DNA
<213> Artificial Sequence

<220>
<221> source
<222> 1..21
<223> /mol_type="unassigned DNA"
      /note="Forward Primer"
      /organism="Artificial Sequence"

<400> 27
tctgacattc caacctttc g                                                  21


<210> 28
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 28
acaccagaac ctcagccaac                                                   20


<210> 29
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 29
ctgatggggt tgtgaggaac                                                   20


<210> 30
<211> 20
<212> DNA
<213> Artificial Sequence

<220>
<221> source

<222> 1..20
<223> /mol_type="unassigned DNA"
      /note="Reverse Primer"
      /organism="Artificial Sequence"

<400> 30
gccttggaca tcatttggaa                                                   20


<210> 31
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 31
acagaatggc cagagtgacc                                                   20


<210> 32
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 32
ttacttgccc tcatcccttg                                                   20


<210> 33
<211> 23
<212> DNA
<213> Artificial Sequence

<220>
<221> source
<222> 1..23
<223> /mol_type="unassigned DNA"
      /note="Forward Primer"
      /organism="Artificial Sequence"

<400> 33
cgtgtcagat gatcaattgt gtt                                               23


<210> 34
<211> 21
<212> DNA
<213> Artificial Sequence

<220>
<221> source
<222> 1..21
<223> /mol_type="unassigned DNA"
      /note="Reverse Primer"
      /organism="Artificial Sequence"

<400> 34

aggaagggtg cttattccaa a                                                21

<210> 35
<211> 23
<212> DNA
<213> Artificial Sequence

<220>
<221> source
<222> 1..23
<223> /mol_type="unassigned DNA"
      /note="Forward Primer"
      /organism="Artificial Sequence"

<400> 35
gtgtggcaaa gtaatgtcag aga                                              23

<210> 36
<211> 22
<212> DNA
<213> Artificial Sequence

<220>
<221> source
<222> 1..22
<223> /mol_type="unassigned DNA"
      /note="Reverse Primer"
      /organism="Artificial Sequence"

<400> 36
ggaagaacac agaagacgtt ga                                               22

<210> 37
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 37
tccctccctc cctctctcta                                                  20

<210> 38
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 38
gacatccata cgcctccact                                                  20

<210> 39
<211> 20
<212> DNA

<213> Artificial Sequence

<220>
<221> source
<222> 1..20
<223> /mol_type="unassigned DNA"
      /note="Forward Primer"
      /organism="Artificial Sequence"

<400> 39
gctattgcac caggcatgta                                                   20


<210> 40
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 40
caggtgggtc tctccatgtt                                                   20


<210> 41
<211> 23
<212> DNA
<213> Artificial Sequence

<220>
<221> source
<222> 1..23
<223> /mol_type="unassigned DNA"
      /note="Forward Primer"
      /organism="Artificial Sequence"

<400> 41
tgaaactgta tatggcattg ctt                                               23


<210> 42
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 42
gcatttgcag ttccagaaaa                                                   20


<210> 43
<211> 20
<212> DNA
<213> Artificial Sequence

<220>
<221> source
<222> 1..20
<223> /mol_type="unassigned DNA"

/note="Forward Primer"
      /organism="Artificial Sequence"

<400> 43
cgttctacgt cggctatggt                                                   20


<210> 44
<211> 23
<212> DNA
<213> Artificial Sequence

<220>
<221> source
<222> 1..23
<223> /mol_type="unassigned DNA"
      /note="Reverse Primer"
      /organism="Artificial Sequence"

<400> 44
cgagaaaaca ctctaacctc caa                                               23


<210> 45
<211> 21
<212> DNA
<213> Artificial Sequence

<220>
<221> source
<222> 1..21
<223> /mol_type="unassigned DNA"
      /note="Forward Primer"
      /organism="Artificial Sequence"

<400> 45
tgcatgcagt ctgcttcata g                                                 21


<210> 46
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 46
ggaaaagtgc aacgaggaaa                                                   20


<210> 47
<211> 22
<212> DNA
<213> Artificial Sequence

<220>
<221> source
<222> 1..22
<223> /mol_type="unassigned DNA"
      /note="Forward Primer"
      /organism="Artificial Sequence"

<400> 47
cttcttttg gtcccctttc tt                                                 22

<210> 48
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 48
cagtggcaaa agcaagcata                                                   20


<210> 49
<211> 19
<212> DNA
<213> Artificial Sequence

<220>
<221> source
<222> 1..19
<223> /mol_type="unassigned DNA"
      /note="Forward Primer"
      /organism="Artificial Sequence"

<400> 49
aggggacaca gagccaaac                                                    19


<210> 50
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 50
cccagacccc tcctacactt                                                   20


<210> 51
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 51
agattgtgct gctccactcc                                                   20


<210> 52
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 52
taggcaagat gctggctcat                                                   20


<210> 53
<211> 21
<212> DNA
<213> Artificial Sequence

<220>
<221> source
<222> 1..21
<223> /mol_type="unassigned DNA"
      /note="Forward Primer"
      /organism="Artificial Sequence"

<400> 53
tgtgacaggg ttctgaaaag c                                                 21


<210> 54
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 54
aggggaaatc aggctaggaa                                                   20


<210> 55
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 55
ccctccccac aattcttttt                                                   20


<210> 56
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 56
agcgcctgga atttttctct                                                   20


<210> 57
<211> 21
<212> DNA
<213> Artificial Sequence

<220>
<221> source
<222> 1..21
<223> /mol_type="unassigned DNA"
      /note="Forward Primer"
      /organism="Artificial Sequence"

<400> 57
tgctctcatt tttctccttg c                                                 21


<210> 58
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 58
gaaaaatggc aaggaagctg                                                   20


<210> 59
<211> 19
<212> DNA
<213> Artificial Sequence

<220>
<221> source
<222> 1..19
<223> /mol_type="unassigned DNA"
      /note="Forward Primer"
      /organism="Artificial Sequence"

<400> 59
gggctgatcc atgcctagt                                                    19


<210> 60
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 60
ggctgaaaca ggaggactga                                                   20


<210> 61

<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 61
gctcctgcct ttctgtcagt                                                   20


<210> 62
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 62
gctgggatta taggcgtgag                                                   20


<210> 63
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 63
tcagctctca ggcatgtgtt                                                   20


<210> 64
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 64
tatatgcacc cccaagtgaa                                                   20


<210> 65
<211> 20
<212> DNA
<213> Artificial Sequence

<220>
<221> source

<222> 1..20
<223> /mol_type="unassigned DNA"
      /note="Forward Primer"
      /organism="Artificial Sequence"

<400> 65
tccctcgctt gatggtaact                                                   20


<210> 66
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 66
atgtccctgg ctctgtctgt                                                   20


<210> 67
<211> 21
<212> DNA
<213> Artificial Sequence

<220>
<221> source
<222> 1..21
<223> /mol_type="unassigned DNA"
      /note="Forward Primer"
      /organism="Artificial Sequence"

<400> 67
gctctggttt cactgtggtt c                                                 21


<210> 68
<211> 22
<212> DNA
<213> Artificial Sequence

<220>
<221> source
<222> 1..22
<223> /mol_type="unassigned DNA"
      /note="Reverse Primer"
      /organism="Artificial Sequence"

<400> 68
caaggctctc tcttaagcaa cc                                                22


<210> 69
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 69

gggaatgatg ctcagtggtt                                                    20

<210> 70
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 70
ctgggtgctg gagagtgttc                                                    20

<210> 71
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 71
cgggatatag gtgagcagga                                                    20

<210> 72
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 72
ttctggccct tgtgtcattt                                                    20

<210> 73
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 73
gctccagttt gcctttttca                                                    20

<210> 74
<211> 20
<212> DNA

<213> Artificial Sequence

<220>
<221> source
<222> 1..20
<223> /mol_type="unassigned DNA"
      /note="Reverse Primer"
      /organism="Artificial Sequence"

<400> 74
   ctccctgtct acccctttcc                                                    20


<210> 75
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 75
ttttccccct gttacgtgag                                                   20


<210> 76
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 76
gccaagtgcc tttttctctg                                                   20


<210> 77
<211> 23
<212> DNA
<213> Artificial Sequence

<220>
<221> source
<222> 1..23
<223> /mol_type="unassigned DNA"
      /note="Forward Primer"
      /organism="Artificial Sequence"

<400> 77
tgagcaatca tctgttgttt tca                                               23


<210> 78
<211> 22
<212> DNA
<213> Artificial Sequence

<220>
<221> source
<222> 1..22
<223> /mol_type="unassigned DNA"

/note="Reverse Primer"
      /organism="Artificial Sequence"

<400> 78
tgaaaaccca cttccacata ca                                              22


<210> 79
<211> 21
<212> DNA
<213> Artificial Sequence

<220>
<221> source
<222> 1..21
<223> /mol_type="unassigned DNA"
      /note="Forward Primer"
      /organism="Artificial Sequence"

<400> 79
gagcttcaaa cagatggagg a                                               21


<210> 80
<211> 21
<212> DNA
<213> Artificial Sequence

<220>
<221> source
<222> 1..21
<223> /mol_type="unassigned DNA"
      /note="Reverse Primer"
      /organism="Artificial Sequence"

<400> 80
gtggccataa ttcaggacaa t                                               21


<210> 81
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 81
gggacctgcc ctttattttg                                                 20


<210> 82
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 82
atccaaagcc cactctctga                                                 20

<210> 83
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 83
accaccctgt gttttccttg                                                   20


<210> 84
<211> 18
<212> DNA
<213> Artificial Sequence

<220>
<221> source
<222> 1..18
<223> /mol_type="unassigned DNA"
      /note="Reverse Primer"
      /organism="Artificial Sequence"

<400> 84
aggccctgca tctcacac                                                     18


<210> 85
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 85
tgatggcctt gtctcctctt                                                   20


<210> 86
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 86
ggggacaagt cagtcttcca                                                   20


<210> 87
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 87
tctgagcaag ctgctgtcat                                                   20


<210> 88
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 88
gcgatctgtt ttaccctcca                                                   20


<210> 89
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 89
tgcttcctcc tcctgtgact                                                   20


<210> 90
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 90
cttagttgca gcccaattcc                                                   20


<210> 91
<211> 27
<212> DNA
<213> Artificial Sequence

<220>
<221> source
<222> 1..27
<223> /mol_type="unassigned DNA"
      /note="Forward Primer"
      /organism="Artificial Sequence"

<400> 91
agtacagctt atttactgtg ttttcct                                          27


<210> 92
<211> 19
<212> DNA
<213> Artificial Sequence

<220>
<221> source
<222> 1..19
<223> /mol_type="unassigned DNA"
      /note="Reverse Primer"
      /organism="Artificial Sequence"

<400> 92
agctttggac ttcagcctc                                                   19


<210> 93
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 93
ttgctttgct gacctgacac                                                  20


<210> 94
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 94
acttgcccaa gctctcaaaa                                                  20


<210> 95
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 95
tgggagtccc ttctttttca                                                  20


<210> 96

<211> 18
<212> DNA
<213> Artificial Sequence

<220>
<221> source
<222> 1..18
<223> /mol_type="unassigned DNA"
      /note="Reverse Primer"
      /organism="Artificial Sequence"

<400> 96
gcccctcttg ctgtctca                                                     18


<210> 97
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 97
cctgtcactc ccttgtctcc                                                   20


<210> 98
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 98
gaaaaacact tgcccctgtg                                                   20


<210> 99
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 99
tccttctctc ccctccactt                                                   20


<210> 100
<211> 20
<212> DNA
<213> Artificial Sequence

<220>
<221> source

<222> 1..20
<223> /mol_type="unassigned DNA"
      /note="Reverse Primer"
      /organism="Artificial Sequence"

<400> 100
accttgtctg ggttgagcat                                                   20


<210> 101
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 101
ccctcctgtg tgctgacata                                                   20


<210> 102
<211> 18
<212> DNA
<213> Artificial Sequence

<220>
<221> source
<222> 1..18
<223> /mol_type="unassigned DNA"
      /note="Reverse Primer"
      /organism="Artificial Sequence"

<400> 102
gagccaaagg cctgacaa                                                     18


<210> 103
<211> 21
<212> DNA
<213> Artificial Sequence

<220>
<221> source
<222> 1..21
<223> /mol_type="unassigned DNA"
      /note="Forward Primer"
      /organism="Artificial Sequence"

<400> 103
gggttttctc cttgggaagt a                                                 21


<210> 104
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 104

cggggtctta ctggttgaga                                                    20


<210> 105
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 105
acagaagact ggcccagaga                                                    20


<210> 106
<211> 21
<212> DNA
<213> Artificial Sequence

<220>
<221> source
<222> 1..21
<223> /mol_type="unassigned DNA"
      /note="Reverse Primer"
      /organism="Artificial Sequence"

<400> 106
ttcccactct tcacacttgc t                                                  21


<210> 107
<211> 20
<212> DNA
<213> Artificial Sequence

<220>
<221> source
<222> 1..20
<223> /mol_type="unassigned DNA"
      /note="Forward Primer"
      /organism="Artificial Sequence"

<400> 107
gcatgtcttc ctgtccttcc                                                    20


<210> 108
<211> 20
<212> DNA
<213> Artificial Sequence

<220>
<221> source
<222> 1..20
<223> /mol_type="unassigned DNA"
      /note="Reverse Primer"
      /organism="Artificial Sequence"

<400> 108
aggtggttct gcagccatag                                                    20


<210> 109
<211> 19
<212> DNA

<213> Artificial Sequence

<220>
<221> source
<222> 1..19
<223> /mol_type="unassigned DNA"
      /note="Forward Primer"
      /organism="Artificial Sequence"

<400> 109
cgctcccctt tctcttcag                                                    19


<210> 110
<211> 18
<212> DNA
<213> Artificial Sequence

<220>
<221> source
<222> 1..18
<223> /mol_type="unassigned DNA"
      /note="Reverse Primer"
      /organism="Artificial Sequence"

<400> 110
caccttcccc cagcacag                                                     18


<210> 111
<211> 21
<212> DNA
<213> Artificial Sequence

<220>
<221> source
<222> 1..21
<223> /mol_type="unassigned DNA"
      /note="Forward Primer"
      /organism="Artificial Sequence"

<400> 111
agtaggcgct tcaggcttag g                                                 21


<210> 112
<211> 21
<212> DNA
<213> Artificial Sequence

<220>
<221> source
<222> 1..21
<223> /mol_type="unassigned DNA"
      /note="Reverse Primer"
      /organism="Artificial Sequence"

<400> 112
gattccaggc aggctctagg a                                                 21